# EUROPEAN PATENT APPLICATION

(11) **EP 3 951 374 A1**
(43) Date of publication of application: **09.02.2022**
(21) Application number: 20382721.7
(22) Date of filing: 03.08.2020
(51) Int. Cl.: G01N 27/27, B01L 3/00, G01N 33/49, G01N 33/543

(54) **BIOSENSOR SYSTEM FOR MULTIPLEXED DETECTION OF BIOMARKERS**

(71) Applicant: Consejo Superior de Investigaciones Científicas (CSIC), 28006 Madrid (ES)
(72) Inventor: FERNÁNDEZ SÁNCHEZ, César, 28006 Madrid (ES); BALDI COLL, Antoni, 28006 Madrid (ES); GUTIÉRREZ CAPITÁN, Manuel, 28006 Madrid (ES)
(74) Representative: Hoffmann Eitle

(57) **Abstract**

The invention refers to a biosensor system for quick and multiplexed detection of biomarkers present in biological fluids. The biosensor system comprises a reusable array of at least two individual electrochemical cells (1a,1b,1c,1d,1e) coupled to a disposable fluidic component. Each cell can be addressed individually. The array includes a set of working electrodes (2a,2b,2c,2d) and at least one shared counter/reference electrode (5) in common for all the electrochemical cells, such that each electrochemical cell includes one working electrode and the shared counter/reference electrode. Preferably, the system includes a disposable paper component (8) having a reactive microfluidic component distributed in fluidic channels (9), isolated by hydrophobic barriers (10). The paper component (8) is operatively aligned with the array of electrochemical cells for the electrochemical detection by means of a polymeric cartridge. The multiplexed biosensor system features a reduced size, and that allows reduction of analysis costs and material waste.

## Description

### Field and object of the invention

The present invention refers in general to biosensor systems for quick and multiplexed detection of biomarkers present in biological fluids.

An object of the invention is to provide biosensor systems of reduced size, and that additionally reduce analysis costs and material waste.

An additional object of the invention is to provide a biosensor system that simplifies scalability of an array of electrochemical cells to be applied, for example for ELISA assays.

An additional object of the invention if to provide a multiplexed biosensor system that combines a reusable array of electrochemical cells and a disposable microfluidic component.

### Background of the invention

There are many different types of biosensors to electrochemically analyze biomarkers contained in biological fluids like: blood, serum, sweat, tear, urine, saliva, sputum, nasopharyngeal and oropharyngeal specimens. A biomarker is a substance used as an indicator of a normal biological process, disease or response to a drug treatment.

Measuring enzyme activity for a specific substance contained in a biofluid with good reproducibility, is important for an electrochemical biosensor based on enzymatic detection approach such as, for example, glucose sensor, uric acid sensor, protein sensor, or a DNA sensor for clinical chemical tests.

Some known biosensors combine a fluidic component in paper (cellulose or nitrocellulose) and a matrix or array of miniaturized electrochemical cells, that are usually manufactured using screen-printing techniques. Typically, both, the fluidic component and the array of transducers are designed as disposable components, such that after a test has been performed, both components are discarded. Therefore, these type of clinical analysis or tests, generate a significant amount of waste.

Furthermore, one of the most important limitations when designing compact electrochemical cells in a planar configuration is the space on the substrate occupied by electrical tracks and associated contact pads of electrodes operating independently. For a conventional three-electrode electrochemical cell configuration, (working, counter and reference electrodes WE, CE, and RE), the number of required connections when fabricating electrochemical cell arrays is three times the number of cells, which greatly limit the size of the array that can be defined on one substrate, and increase manufacturing complexity and cost.

Different approaches to overcome this limitation have been proposed, generally based on the use of grid-patterned electrodes inserted in specially designed microfluidic devices to enabling multiplex detection, or using a travelling light pointer to form transient working electrodes or sharing working electrode connections and multiple fluid compartments. Even though a high density of working electrodes is achieved, external or on-chip counter electrodes and/or reference electrodes are shared among many working electrodes immersed in the same liquid pool.

This hampers the use of fully addressable electrochemical cells that could be in contact with solutions of different composition, as required for example for electrochemical ELISA assays carried out in conventional 96-well microtiter plates.

### Summary of the invention

The present invention is defined in the attached independent claim, and satisfactorily solves the shortcomings of the prior art, by providing a biosensor system of reduced size for the multiplexed and quick detection of biomarkers in biological fluids like for example: blood, serum, sweat, tear, urine, saliva, sputum, nasopharyngeal and oropharyngeal specimens.

The biosensor system of the invention comprises: a reusable array comprising at least two individual electrochemical cells that are individually addressable, a disposable fluidic component, and a cartridge to integrate and align the array and the microfluidic component.

The at least two individual electrochemical cells, operate in isolated liquid wells patterned in a polymeric structure, and arranged in correspondence with the electrochemical cells for the electrochemical detection of individual samples contained in each liquid well.

The system further comprises a set of working electrodes and at least one counter / reference electrode common to all the electrochemical cells, such that each electrochemical cell includes one working electrode and a defined area of the shared counter/reference electrode.

The counter / reference electrode is used as counter electrode and at the same time as reference electrode for the electrochemical analysis.

In a first preferred embodiment, the working electrodes are aligned in a longitudinal straight direction, and the shared counter / reference electrode is a straight track, such that the working electrodes are adjacent to one side of the shared counter/reference electrode. In addition, the longitudinal straight direction of the working electrodes alignment, is parallel to the counter / reference electrode (two-electrode configuration).

In a second preferred embodiment, the biosensor system comprises two shared electrodes, namely a counter electrode and reference electrode, such that the counter and reference electrodes are common electrodes for all the working electrodes, that is, the counter and reference electrodes are shared by all the electrochemical cells (three-electrode configuration).

Preferably, the counter electrode and the reference electrode are straight tracks parallel to each other, and parallel to the longitudinal alignment direction of the working electrodes. Furthermore, the longitudinal alignment direction of the working electrodes, is placed in between the counter electrode and the reference electrode.

Preferably, the liquid wells are also aligned in a straight longitudinal direction, following the parallel arrangement of the counter / reference electrode of the first embodiment, and that of the counter and reference electrodes of the second embodiment, and to the longitudinal alignment direction of the working electrodes.

The configuration of the electrochemical cells allows multi-parametric or multisample analysis, because a desired number of electrochemical cells can be included in a transducer chip design. Using the same number of isolated liquid wells, the electrochemical cells are individually addressable, that is, a determination can be made in each electrochemical cell, either of the same biomarker in different samples, or different biomarkers in the same sample.

In both embodiments described above, the system further comprises a set of connection pads, and a set of connection tracks connecting the working electrodes, shared counter/reference electrode, or shared counter electrode and a shared reference electrode, with the connection pads. Preferably, a part of each connection track is parallel to the counter/reference electrode, and to the shared counter and shared reference electrodes.

In a preferred embodiment, the system can further comprise a disposable fluidic component, preferably made of paper (cellulose or nitrocellulose), for analysis under capillary flow conditions, where distributed fluidic channels are defined and isolated by hydrophobic barriers. The fluidic component formed as a substrate, is operatively couplable with the array of electrochemical cells, for performing electrochemical detection.

Therefore, taking into account the above-described embodiment, the system of the invention is based on the combination of a fluidic component and a matrix or array of electrochemical cells, wherein the fluidic component is discarded after each analysis, and the electrochemical cell component is a reusable part of the device, so that, less waste is generated and the cost per analysis is reduced. This represents a difference with the usual electrochemical tests used in biological measurements, where both paper strip and electrochemical cell are disposable.

The system additionally comprises a cartridge having top and bottom parts couplable to each other, where a chip substrate is enclosed comprising the connection pads and the electrodes to configure the array of electrochemical cells.

Preferably, the chip substrate is generally rectangular and the connection pads are grouped in a reticular matrix arrangement and formed adjacent to one short side of the substrate. A set of spring-loaded connectors is provided for their connection with the electrode contact pads of the electrochemical cells.

The disposable fluidic component can be inserted in the cartridge and pressed between the two parts and operatively coupled with the array of electrochemical cell.

The fluidic channels have the form of strips, such that when the disposable fluidic component is coupled with the electrochemical cells, the fluidic channels are transversally arranged with respect to the shared counter and reference electrodes and the working electrodes of each electrochemical cell.

### Brief description of the drawings

Preferred embodiments of the invention are henceforth described with reference to the accompanying drawings, wherein:
Figure 1.- shows a top plan view of a first implementation of the array of electrochemical cells according to the invention, that includes a layout of a two-electrode configuration.
Figure 2.- shows a top plan view of a second implementation of the array of electrochemical cells, that includes a layout of three-electrode configuration.
Figure 3.- shows an exploded view of a polymeric cartridge defining liquids wells.
Figure 4.- shows in a top plan view, a fluidic component (Figure A), and the arrangement of the fluidic component coupled with the array of electrochemical cells (Figure B) and an absorbent pad overlapping the channels of the fluidic component.
Figure 5.- shows in plan view the layout of the component once coated with vinyl layers for better alignment with the array of electrochemical cells. Figure A is a plan view of the upper side, Figure B is a plan view of the bottom side, and Figure C is a top plan view of the component coupled with the array of electrochemical cells.
Figure 6.- shows in Figure A a top plan view of the layout of a device packaging to easily couple and align the fluidic component with the array of electrochemical cells, and in Figure B an exploded view of the packaging components including the clamping structures used por applying a constant pressure between the top and bottom parts of the cartridge.
Figure 7.- shows a graph of a cyclic voltammogram recorded with one electrochemical cell in a 0.1 M TRIS·HCl buffer solution pH 9.8 containing 1 mM BQ electroactive species. Scan rate= 50 mV/s.

### Preferred embodiment of the invention

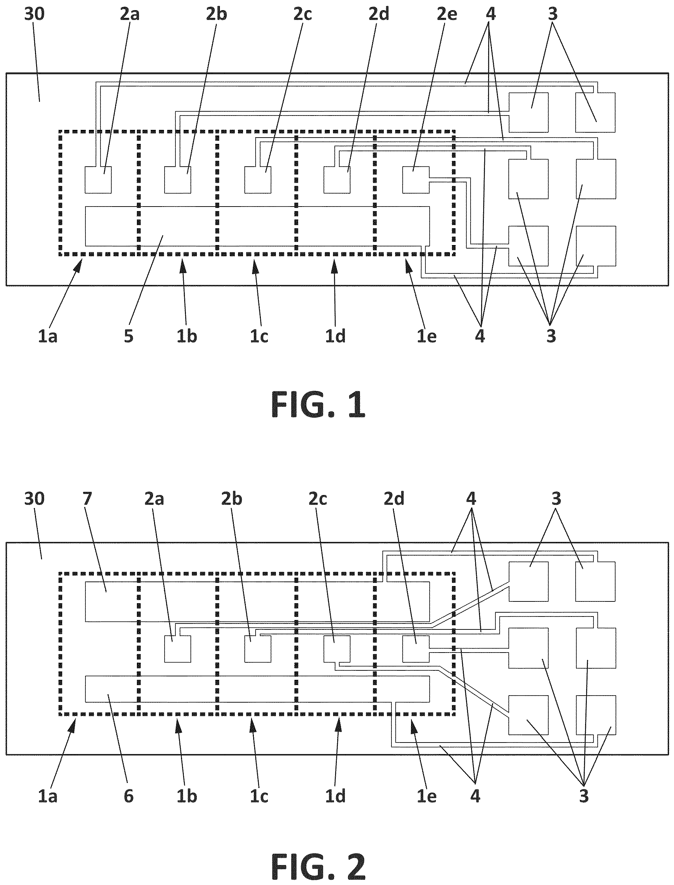

**Figure 1** shows a first preferred implementation of the layout of the electrodes, connection pads and connection tracks formed on a surface of a substrate (30) to produce an array of electrochemical cells (1a,1b,1c,1d,1e) in a two-electrode configuration. A plurality of working electrodes (2a,2b,2c,2d,2e) are aligned along a straight longitudinal direction, and one counter/reference electrode (5) is provided common for all the working electrodes (2a,2b,2c,2d,2e). The counter/reference electrode (5) is a straight track and the working electrodes are arranged adjacent to one side of the shared counter/reference electrode (5), in a way that the longitudinal direction of alignment of the working electrodes, is parallel to the counter/reference electrode (5).

The substrate (30) is rectangular and includes a set of connection pads (3) that are placed adjacent to one short side of the substrate (30) as shown in **Figure 1**. The connection pads (3) are grouped in a reticular matrix arrangement for their connection with a group of spring-loaded connectors, as it will be explained later on.

A plurality of the connection tracks (4) connect individually each working electrode (2a,2b,2c,2d,2e) and the counter/reference electrode (5), each with one connection pad (3). As represented in **Figure 1**, the connection tracks run longitudinally in the substrate (30), in a way that a part of each connection track is parallel to the shared counter / reference electrode (5).

As it can be noted in **Figure 1**, with this layout, where the counter/reference electrode is common to all the working electrodes, the number of connection tracks and connection pads, is significantly reduced so that the substrate (30) features a compact design.

Each electrochemical cell (1a,1b,1c,1d,1e) comprises one working electrode (2a,2b,2c,2d,2e) and the shared counter/reference electrode (5), thus, in the embodiment of **Figure 1** there are five electrochemical cells that could be integrated in a single device.

**Figure 2** shows an alternative electrode layout on the substrate (30) for a three-electrode cell configuration. In this case, there are four working electrodes (2a,2b,2c,2d), a reference electrode (6), a counter electrode (7), a group of connection pads (3), and a plurality of connection tracks (4) connecting individually the working electrodes with the connection pads (3). The reference and the counter electrodes (6,7), are common for all the working electrodes, so they are shared by all the electrochemical cells (1a,1b,1c,1d,1e).

The working electrodes (2a,2b,2c,2d) are also aligned, and the reference and counter electrodes (6,7) are straight tracks parallel to each other, and parallel to the longitudinal alignment direction of the working electrodes. The working electrodes are arranged in between the reference and the counter electrodes (6,7). Several connection tracks (4) connect individually all the electrodes (2,6,7) with the connection pads, and have a part parallel to the reference and the counter electrodes (6,7).

In this case, each electrochemical cell (1a,1b,1c,1d,1e) includes a working electrode (2a,2b,2c,2d) and a defined area of the reference and counter electrodes (6,7).

The embodiment of **Figure 2** includes four electrochemical cells (1b,1c,1d,1e) in a three-electrode configuration for the simultaneous measurement of biomarkers, and an additional cell (1a) including only two electrodes (the counter and the reference electrodes), which is used for a reference channel.

In both implementations described above, all the electrodes of the electrochemical cells can be manufactured by standard photolithographic techniques, by patterning gold or platinum electrodes on silicon substrates.

As it can be observed in **Figures 1** and **2**, the fact that all electrochemical cells shared counter/reference electrode (two-electrode cells) or a common counter and reference electrodes (three-electrode cells), has the advantage that only six contact pads are required, which greatly reduce the number of contact pads for connecting externally all the cells.

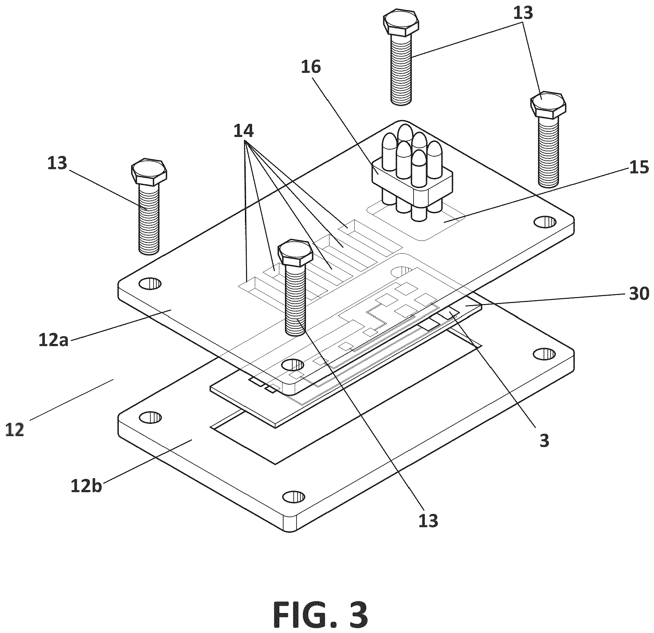

As represented in **Figure 3**, the array of electrochemical cells (1a,1b,1c,1d,1e) can be implemented in a cartridge (12) having top and bottom parts (12a,12b) couplable to each other, for example by means of screws (13) or other type of clamping means, so that the array is enclosed between the top and bottom parts (12a,12b). The top part (12a) has several windows that configure isolated liquid wells (14), that are distributed such that each well (14) is placed right on top of an electrochemical cell. In this way, the electrochemical cells (1a,1b,1c,1d,1e) are fully addressable individually.

In a practical implementation of the invention, the capacity of each well (14) is around 75-µL.

In the preferred embodiment represented in the **Figure 3**, the wells (14) are aligned in a straight longitudinal direction, that is parallel to the shared reference and counter electrodes, and parallel to the longitudinal alignment direction of the working electrodes.

The top part (12a) has an additional window (15) for the insertion of a set of spring-loaded connectors (16), located right on the connection pads (3), in a way that when the cartridge is assembled, each connector (16) would be connected to a connection pad (3). The connectors (16) serve to connect the electrochemical cells with an external measurement device.

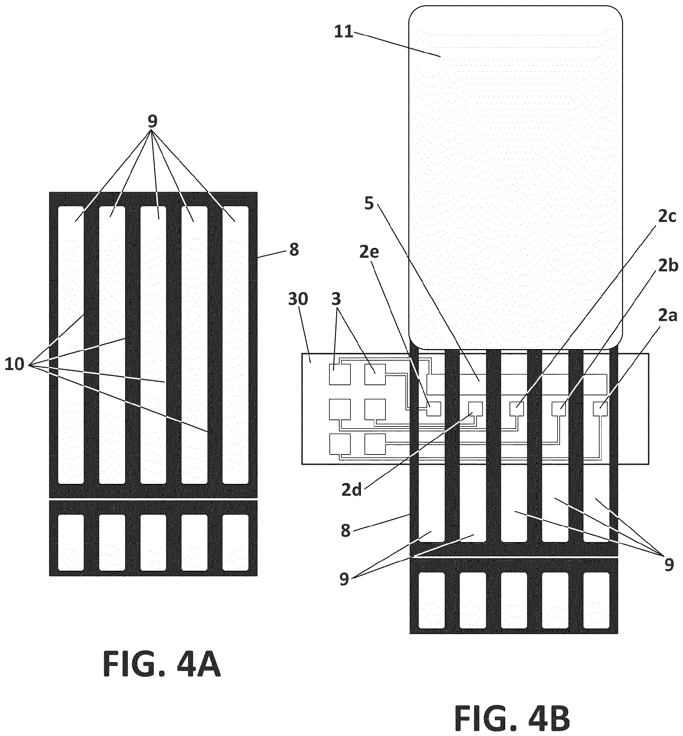

As shown in **Figure 4A****,** the biosensor system includes a disposable paper component (8) in the form of a substrate being a reactive fluidic component comprising fluidic channels (9) arranged in a way that the channels (9) are isolated by hydrophobic barriers (10).

In this preferred embodiment, the fluidic channels (9) have the form of straight strips. However, in other preferred embodiment, the fluidic channels (9) might have other configurations suitable for each application.

The two implementations of **Figure 1** and **2** are designed such that they could be easily coupled to the paper microfluidic component (8). In addition, the design in both implementations features a reduced size as the overall dimensions of the device are 25x8 mm².

As represented in **Figure 4B**, the paper component (8) is operatively couplable with the array of electrochemical cells (1a,1b,1c,1d,1e) formed on the substrate (30) for the electrochemical detection, such that, each channel (9) is aligned over an electrochemical cell (1a,1b,1c,1d,1e), that is, on a working electrode (2a,2b,2c,2d,2e) and on a part of the counter / reference electrode (5).

When the disposable paper component (8) is coupled with the electrochemical cell array, the fluidic channels (9) are transversally arranged, preferably orthogonally, with respect to the counter / reference electrode (5), and with respect to the alignment of the working electrodes (2a,2b,2c,2d,2e).

The system includes an absorbent pad (11) overlapped and in contact with the channels (9) of the paper component.

Printed hydrophobic walls needed for the design of the channels, showed good resolution. Having a very lost-cost easy-to-manufacture disposable paper component, reduces cost per analysis required for Point-Of-Care devices.

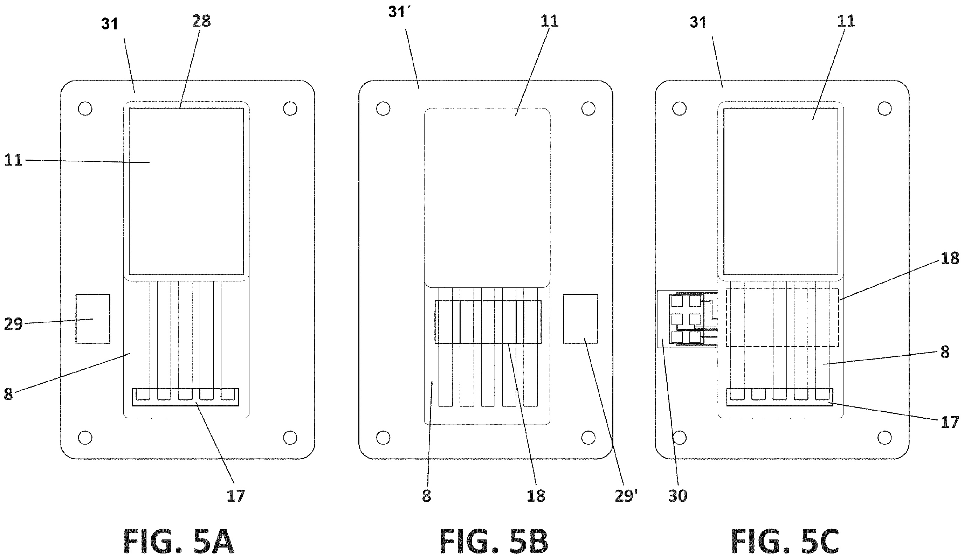

**Figure 5** shows the layout of the paper component (8) sandwiched between vinyl layers (31,31') for its easy alignment with the array of electrochemical cells. The vinyl layers (31,31') are patterned using a blade plotter, so that several areas of the paper component (8) are exposed.

As shown in **Figure 5A** a first window (17) is opened at a top surface for receiving samples, that leaves open the initial part of the paper channels for the sample addition. A second window (28) is opened at the sink area to allow liquid evaporation once it has reached the absorbent pad (11). A third window (18) is opened at the back side of the paper component (8) to provide access to the sensing areas of the same **(****Figure 5B****)**, so that the paper component and the electrochemical cells can get in contact.

On both vinyl layers (31,31'), additional windows (29,29') are opened for the connector of the electrode array to be placed over the electrode contact pads. **Figure 5C** shows the relative positions of all the above-described windows.

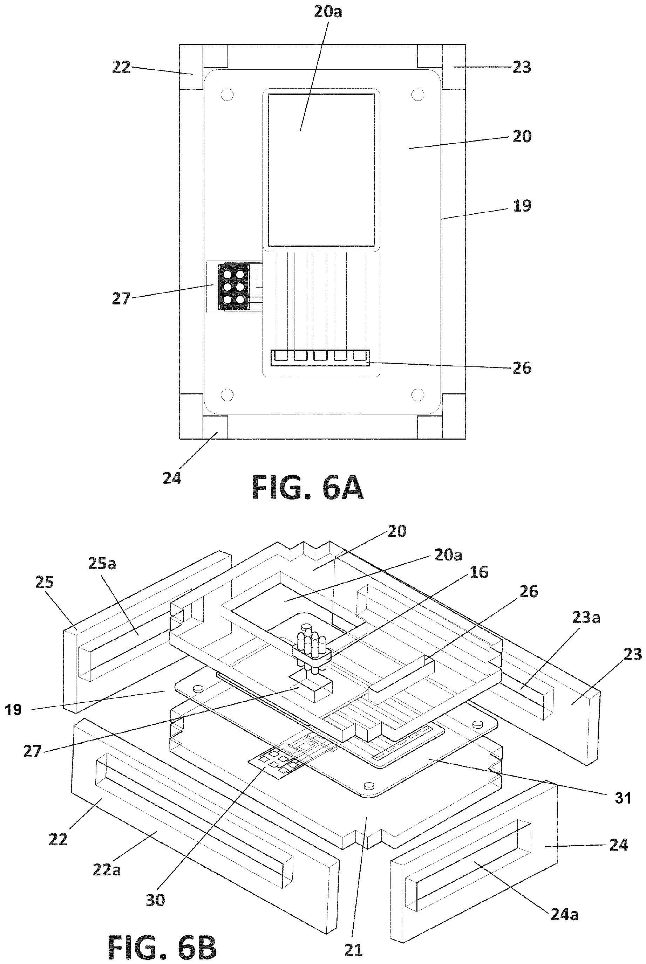

In practice, the coupling of the array of electrochemical cells and the disposable paper fluidic component (8) is done by means of a packaging cartridge (19) including top and bottom parts (20.21) and pressed together with clamping means, as shown in **Figure 6**.

**Figure 6** illustrates the packaging cartridge (19) that integrates the packaged paper fluidic component (8) and the array of electrochemical cells (1a,1b,1c,1d,1e). The packaging cartridge (19) comprises top and bottom parts (20,21), and clamping means configured in this example as first and second large lateral walls (22,23), and first and second short lateral walls (24,25), such that, the lateral walls retain top and bottom parts (20,21) pressed against each other and keep both, the paper component and electrochemical cell array, robustly aligned during measurements.

The top part (20) has several windows to provide access to the packaging cartridge (19) interior, in particular it has: an evaporation window (20a) that is placed over the absorbent pad (11), and serves as sink area evaporation window, a sample access window (26) that is placed right over the first window (17) of the paper component (8), and a connector window (27) for receiving the spring-loaded connectors (16).

The bottom part (21) can integrate five 2-mm diameter Nd magnets (not shown) located right before the detection area of the electrode array, and aligned with the fluidic channels (9) so that, when working with magnetic nanoparticles, these flow on the paper and can be trapped and accumulated before carrying out the measurements.

As shown specially in **Figure 6B**, top and bottom parts (20,21) are flat and generally rectangular and have cut-out corners, that conform protruding four edges in both covers.

Clamping means are provided for package assembly, wherein the clamping means are adapted to attach and press together top and bottom parts (20,21). In this exemplary embodiment, the clamping means are embodied as first and second large lateral walls (22,23) having respective windows (22a,23a), and first and second short lateral walls (24,25) have respective windows (24a,25a). The large lateral edges of the top and bottom covers (20,21) can be inserted respectively in the windows (22a,23a) of the large lateral walls, and the short edges of the top and bottom covers (20,21) can be inserted in the windows (24a,25a) of the two lateral short walls (24,25), conforming thereby a packaging cartridge (19) as the one shown in **Figure 6A**.

Since the lateral walls are used as clamping means, the packaging cartridge (19) can be easily assembled and disassembled to get access to both the electrochemical cell array and the paper component so that the paper component can be easily removed after one measurement and replace it for a new one and the reusable array of electrochemical cells can be cleaned if necessary and replaced once its life cycle finishes. The packaging cartridge (19) can be manufactured at a low cost, because it does not require mechanical fastening means for its assembly. The package can be made for example by laser cutting of polymethylmethacrylate (PMMA) plates. Other polymeric materials can be used and patterned by injection molding or 3D printing.

In addition, the encapsulation of the paper component (8) with the vinyl layers (31,31') achieves a reproducible alignment between the electrochemical cells and the paper channel that does not rely on double-sided adhesives to bond the paper fluid component to the electrochemical cells as in prior art solutions. The packaging cartridge (19) applies an even pressure on the paper component (8), and ensures a proper contact between the paper channels and all the electrochemical cells in the array.

For the analytical assessment of the proposed electrochemical cell array, several experiments have been carried out with the three-electrode cell array in solutions containing ferrocyanide / ferricyanide redox pair by cyclic voltammetry. It was seen that the electrochemical response of each cell in the array was very similar to the one of an individual cell. Because of the cell configuration and in order to carry out the quasi-simultaneous measurements in all cells of the array, the two species of the redox pair should be present in solution. Working in this way, calibration curves for ferricyanide could be plotted for all cells in the array. The potential difference between the counter and reference electrode was kept very low (below 30 mV) during the timescale of the experiments. Thus, the use of a single counter/reference electrode was feasible and the two-electrode cell array was then characterised.

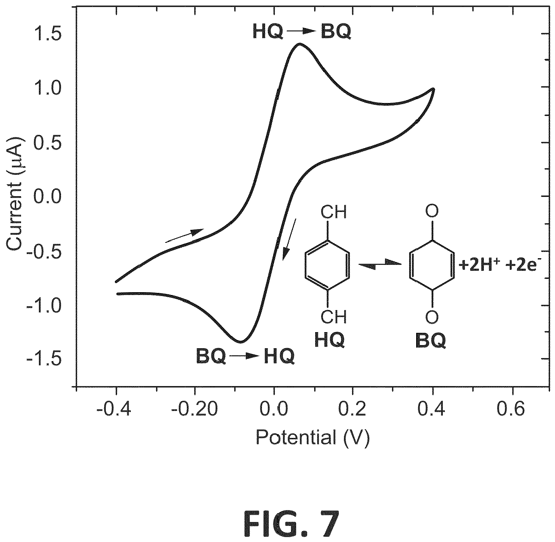

Similar experiments to the ones carried out with the previous array, have been performed and all the two-electrode cells in the array could be calibrated. As an example, the array was characterized by cyclic voltammetry (CV) in solutions containing ferrocyanide/ferricyanide or hydroquinone/benzoquinone (HQ/BQ) reversible redox pairs, as represented in **Figure 7**. One of the species of the redox pair was measured with a linear response in individual cells of the array in the presence of an excess concentration of the redox counterpart.

Using the ferrocyanide / ferricyanide redox pair and as a first approach for measuring the activity of an enzyme label, some tests with Horseradish Peroxidase (HRP) have been carried out using ferrocyanide as the enzyme mediator. This enzyme converted ferrocyanide into ferricyanide product, which was measured by chronoamperometry using the two-electrode cell array. A similar experiment was performed with myeloperoxidase (MPO) but using ferrocene-methanol as a mediator as well as with alkaline phosphatase (ALP) using hydroquinone diphosphate (HQDP) as substrate, and results were highly satisfactory.

The electrochemical detection scheme of the device for the three biomarkers is as follows:
- the two-electrode cell array was implemented and the electrochemical transduction mode is chronoamperometry.
- MPO activity could be detected by using ferrocene-methanol redox substrate.
- HRP label together with ferrocyanide substrate was the alternative chosen for carrying out the electrochemical detection of tumor necrosis factor alpha (TNF-α) and cytokine interleukin-8 (IL-8).

In a practical application of the invention, a silicon chip of 8×25 mm2 is manufactured containing five electrochemical cells, with five working electrodes and a shared counter/pseudoreference electrode for all the cells. The electrodes are made of gold.

The paper component of the Whatman Grade 1 Chromatographic type has 5 different channels. Together with the PMMA packaging, the determination of the concentration IL-8 and TNF-alpha, together with the enzyme MPO in sputum samples has been addressed by a sandwich type magnetoimmunoassay. All three analytes are biomarkers of Chronic Obstructive Pulmonary Disease (COPD) and other inflammatory diseases of the respiratory system.

Magnetic nanoparticles functionalized with primary antibodies are incubated with the sample, and after recognition, a second incubation is performed with a secondary antibody labeled with horseradish peroxidase (HRP) or alkaline phosphate (ALP) enzymes. The conjugated nanoparticles can then be concentrated, conditioned in a suitable solution and run through the paper. A magnet embedded in the bottom part of the PMMA cartridge is capable of trapping them, and finally carrying out the electrochemical detection by adding a solution containing with the enzyme substrates to the paper component.

This same procedure can be performed with different types of analytes, for example proteins, enzymes, viruses or bacteria, as well as DNA or RNA sequences, provided that we have the antibodies to perform the immunoassays or the necessary DNA strands.

Alternatively, the two incubations could be done directly in the device, running the functionalized magnetic nanoparticles on the paper while reacting with labelled antibodies or DNA strands previously deposited on the paper fluidic channels.

The biosensor system of the invention, can be marketed as a point-of-care or point-of-need device.

The system was initially assessed with solutions containing the redox mediators that were used for detecting MPO and the HRP labelled of the immunoassays for TNF-α and IL-8. It was shown that both species could be easily detected by chronoamperometry and that just 5 µL of solution were necessary for carrying out the measurements on each channel.

Then, measurements for the three biomarkers (TNF-α, IL-8 and MPO) were carried out in standard buffered solutions. These involved the following steps: Incubation of the antibody-coated magnetic nanoparticles in solutions containing different biomarker concentrations; washing; incubation of the magnetic nanoparticles in solutions containing the corresponding detection antibodies labelled with HRP for TNF- α and IL-8 assay; washing; casting of 5 µL of the MNP suspension on the paper channels; 2 rinsing steps, each of them with 5 µL rinsing solution; casting of 5 µL of enzyme substrate solutions and incubation for 3 min; final chronoamperometric measurement. The assay time for the incubations is under 90 min.

Other preferred embodiments of the present invention are described in the appended dependent claims and the multiple combinations of those claims.

## Claims

1. A biosensor system for multiplexed detection of biomarkers, the system comprising:
a reusable array comprising at least two individual electrochemical cells (1a,1b,1c,1d,1e), to be individually addressable,
a disposable fluidic component (8),
and a packaging cartridge (19) to integrate and align the array and the fluidic component (8).

2. System according to claim 1, further comprising a set of working electrodes (2a,3b,2c,2d,2e) and at least one shared counter/reference electrode (5) in common for all the electrochemical cells, such that each cell includes one working electrode (2a,3b,2c,2d,2e) and the shared counter/reference electrode (5), and wherein the working electrodes (2a,3b,2c,2d,2e) are aligned in a longitudinal straight direction, and the shared counter/reference electrode (5) is a straight track, and wherein the working electrodes are adjacent to one side of the shared counter/reference electrode (5).

3. System according to claim 2, comprising a shared counter electrode (6) and a shared reference electrode (7), wherein the shared counter and reference electrodes (6,7) are common electrodes for all the electrochemical cells.

4. System according to claim 3, wherein the shared counter electrode (6) and the shared reference electrode (7) are straight tracks parallel to each other, and parallel to the longitudinal alignment direction of the working electrodes (2a,3b,2c,2d,2e), and wherein the working electrodes are aligned and arranged in between the shared counter electrode and a shared reference electrode.

5. System according to any of the preceding claims, wherein each electrochemical cell comprises an isolated liquid well (14), and wherein the wells (14) are aligned in a straight longitudinal direction that is parallel to the shared reference and counter electrodes (6,7), and parallel to the longitudinal alignment direction of the working electrodes (2a,3b,2c,2d,2e).

6. System according to any of the preceding claims, further comprising a set of connection pads (3), and a set of connection tracks (4) connecting the working electrodes (2a,3b,2c,2d,2e), the shared counter/reference electrode (5), or shared counter electrode and a shared reference electrode (6,7), with the connection pads (3), wherein a part of each connection tracks (4) is parallel to the shared counter and shared reference electrodes.

7. System according to any of the preceding claims, further comprising a disposable fluidic component, preferably made of paper (8) having reactive microfluidic channels (9), wherein the channels are isolated by hydrophobic barriers (10), and wherein the paper component (8) is operatively couplable with the array of electrochemical cells for the electrochemical detection.

8. System according to claim 7, wherein the fluidic channels (9) include biocomponents for carrying out the analyses.

9. System according to any of the preceding claims, further comprising a packaging cartridge (12) having top and bottom parts (12a,12b) couplable to each other, and further comprising a chip substrate (30) enclosed inside the cartridge, and wherein the counter (6), reference (7) and working electrodes (2a,3b,2c,2d,2e) are formed on one side of the chip substrate (30), and wherein the liquid wells (14) are formed in one of the parts of the packaging cartridge (12).

10. System according to any of the preceding claims, wherein the disposable fluidic component (8) can be inserted and pressed between the two parts (20,21) of the cartridge (19), and operatively coupled with the array of electrochemical cells.

11. System according to any of the claims 9, wherein the chip substrate (30) is rectangular and the connection pads (3) are grouped in a reticular matrix arrangement and formed adjacent to one short side of the substrate, and wherein the system further comprises spring-loaded contacts (16) connected with the connection pads (3).

12. System according to any of the claims 9 to 11, wherein the fluidic channels (9) have the form of straight strips, such that when the disposable fluidic component (8) is coupled with the transducers, the channels are transversally arranged with respect to the shared counter and reference electrodes (6,7).

13. System according to any of the preceding claims, further comprising clamping means (22,23,24,25) adapted to attach and press together the top and bottom parts (20,21) of the packaging cartridge (19).

14. System according to any of the preceding claims, wherein the top part of the packaging cartridge (20) has an evaporation window (20a) that is placed over the absorbent pad (11), and serves as sink area evaporation window, and a sample access window (26) that is placed right over the first window (17) of the paper component (8), and a connector window (27) for receiving the spring-loaded connectors (16).

15. System also comprising the instrumentation required to carry out the electrochemical detection by different techniques, preferably chronoamperometry, of the multiplexed biosensor.
